# FASCICULE DE BREVET EUROPEEN

(11) **EP 2 935 379 B1**
(45) Date de publication et mention de la délivrance du brevet: **02.05.2018**
(21) Numéro de dépôt: 13803179.4
(22) Date de dépôt: 26.11.2013
(51) Int. Cl.: C08F 265/10, A61Q 19/00, A61K 8/91, A61K 47/32, A61Q 5/12, A61Q 5/06

(54) **NOUVEAUX POLYMÈRES AMPHOLYTES À CARACTÈRE THERMOSENSIBLE**
NEUARTIGE AMPHOLYTISCHE POLYMERE MIT WÄRMEEMPFINDLICHEM CHARAKTER
NOVEL AMPHOLYTIC POLYMERS HAVING A THERMOSENSITIVE CHARACTER

(30) Priorité: 21.12.2012 FR 1262530
(43) Date de publication de la demande: 28.10.2015
(73) Titulaire: Societe D'Exploitation De Produits Pour Les Industries Chimiques Seppic, 75007 Paris (FR)
(72) Inventeur: MALLO, Paul, 78110 Le Vésinet (FR); BRAUN, Olivier, 81100 Castres (FR)
(74) Mandataire: Laigneau, Amandine
(86) Numéro de dépôt international: PCT/FR2013/052856
(87) Numéro de publication internationale: WO 2014/096595

(56) Documents cités:
- WO-A1-00/40958
- WO-A2-2007/000535
- FR-A1- 2 932 183
- XU WU, YINGJIE QIAO, HUI YANG, JINBEN WANG: "Self-assembly of a series of random copolymers bearing amphiphilic side chains", JOURNAL OF COLLOID AND INTERFACE SCIENCE, vol. 349, no. 2, 2 juin 2010 (2010-06-02), pages 560-564, XP002712329, ISSN: 0021-9797
- OYEWOLE TAYE SALAMI, JOHANN PLANK: "Synthesis, Effectiveness, and Working Mechanism of Humic Acid-{sodium 2-acrylamido-2-methylpropane sulfonate-co-N,N-dimethyl acrylamide-co-acrylic acid} Graft Copolymer as High-Temperature Fluid Loss Additive in Oil Well Cementing", JOURNAL OF APPLIED POLYMER SCIENCE, vol. 126, no. 4, 1 mai 2012 (2012-05-01), pages 1449-1460, XP002712330, ISSN: 0021-8995

## Description

La présente demande de brevet a pour objet de nouveaux copolymères ampholytes thermosensibles, le procédé pour leur préparation et leur utilisation en cosmétique.

La demande de brevet français publiée sous le numéro 2 932 183 décrit des nouveaux polyampholytes réticulés, majoritairement anionique, mais comportant aussi des sites cationiques. Ces polyampholytes conduisent après gonflement dans des fluides aqueux à des microgels, déformables, stables en température, stables mécaniquement, lorsqu'ils sont soumis à un fort cisaillement, et qui s'adsorbent de façon irréversible sur les sites anioniques des roches. Des applications sont ainsi possibles pour le traitement des puits producteurs d'hydrocarbures notamment au niveau de la prévention des venues d'eau.

De tels polyampholytes réticulés majoritairement anioniques mais contenant également de petites proportions de sites cationiques peuvent aussi être également avantageusement utilisés sur la peau et le cuir chevelu compte tenu de leurs bonnes propriétés d'adsorption. Cependant ceux divulgués cette demande de brevet français numéro 2 932 183, ne s'adsorbent pas suffisamment sur la peau ou les cheveux pour être utilisés en cosmétique. Il en est de même pour les polymères thermo-épaississants divulgués dans la demande internationale publiée sous le numéro WO 00/40958.

Il existe aujourd'hui un besoin de polymères ayant possédant de bonnes propriétés d'adsorption et des propriétés épaississantes dépendantes de la température.

C'est pourquoi selon un premier aspect, l'invention a pour objet le procédé tel que défini à la revendication 1.

Parmi les monomères de réticulation comprenant au moins deux double liaisons carbone-carbone, il ya par exemple le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Selon un aspect particulier du procédé tel que défini ci-dessus, dans l'étape e) telle que décrite précédemment, l'agent de réticulation est plus particulièrement mis en oeuvre dans une proportion molaire, supérieure ou égale à 0,01% et inférieure ou égale à 0,25%.

Selon deux aspects particuliers de la présente invention, ledit copolymère en peigne obtenu directement par le procédé tel que défini précédemment est caractérisé en ce que, lorsque ledit squelette polymérique comporte des unités monomériques issues du monomère de formule (I), m est égal à 2 ou à 3 dans ladite formule (I) et/ou R₂ et R₃ représentent chacun un radical méthyle.

Selon ces aspects particuliers copolymère en peigne obtenu directement par le procédé tel que défini précédemment est caractérisé en ce que, lorsque ledit squelette polymérique comporte des unités monomériques issues du monomère de formule (I), celles-ci sont issues du :
- Méthacrylate de diméthyl amino éthyle (dénommé ci-après MADMAE), ou du
- N-[3-(diméthylamino propyl)] acrylamide (dénommé ci-après DMAPAA),

Par monomère cationique, on désigne pour le copolymère en peigne tel que défini précédemment, principalement un monomère comportant une fonction ammonium quaternaire et moins une liaison éthylènique. Un tel monomère est généralement disponible sous forme notamment d'un sel d'acide fort.

Par sel d'acide fort, on désigne plus particulièrement les sulfate, nitrate, sulfonate, phosphate, phosphonate, les halogénures, tels que les bromure, chlorure ou iodure desdits monomères à fonction ammonium quaternaire.

Selon un autre aspect particulier de la présente invention, ledit copolymère en peigne obtenu directement par le procédé tel que défini précédemment est caractérisé en ce que, lorsque ledit squelette polymérique comporte des unités monomériques issues d'un monomère cationique, celles-ci sont issues du monomère cationique de formule (III) :

CH₂=C(R₇)-C(=O)-Y-(CH₂)ᵣ-N⁺(R₈)(R₉)(R₁₀), X⁻ (III)

dans laquelle r représente un nombre compris entre 1 et 4, Y représente O ou NH, R₇ représente un atome d'hydrogène ou un radical méthyle, R₈ et R₉ et R₁₀ identiques ou différents représentent un radical méthyle ou un radical éthyle et l'anion X⁻ un anion choisi parmi les ions bromure ou chlorure.

Selon cet autre aspect particulier, lorsque ledit squelette polymérique comporte des unités monomériques issues du monomère cationique de formule (III), celles-ci sont issues des sels d'ammonium quaternaires suivants :
- Le sel de N,N,N-triméthyl 3-[(2-méthyl 1-oxo 2-propènyl) amino] propanammonium ;
- Le sel de N,N,N-triméthyl 3-[(1-oxo 2-propènyl) amino] propanammonium ;
- Le sel de N,N,N-triméthyl 2-[(1-oxo 2-propènyl) oxy] éthanammonium ; ou
- Le sel de N,N,N-triméthyl 2-[(2-méthyl 1-oxo 2-propènyl) oxy] éthanammonium, et tout particulièrement parmi :
- Le chlorure de N,N,N-triméthyl 3-[(1-oxo 2-propènyl) amino] propanammonium,(dénommé APTAC) ;
- Le chlorure de N,N,N-triméthyl 3-[(2-méthyl 1-oxo 2-propènyl) amino] propanammonium (dénommé MAPTAC) ;
- Le chlorure de N,N,N-triméthyl 2-[(1-oxo 2-propènyl) oxy] éthanammonium (dénommé ADQUAT) ; ou
- Le chlorure de N,N,N-triméthyl 2-[(2-méthyl 1-oxo 2-propènyl) oxy] éthanammonium (dénommé MADQUAT™);
   et de préférence parmi :
- Le chlorure de N,N,N-triméthyl 3-[(1-oxo 2-propènyl) amino] propanammonium, ou
- Le chlorure de N,N,N-triméthyl 2-[(2-méthyl 1-oxo 2-propènyl) oxy] éthanammonium.

Selon un autre aspect particulier de la présente invention, ledit copolymère en peigne directement obtenu par le procédé tel que défini précédemment est caractérisé en ce que lesdits segments latéraux macromonomériques, sont issus de la polymérisation du composé de formule (II) telle que définie précédemment, dans laquelle, R₄ représente un atome d'hydrogène ou un radical méthyle et R₅ un atome d'hydrogène et R₆ représente un radical alkyle comportant de 1 à 3 atomes de carbone éventuellement substitué par un groupe hydroxy,

Selon cet aspect particulier le composé de formule (II) est plus particulièrement choisi parmi le N-méthyl méthacrylamide, le N-éthyl méthacrylamide, le N-propyl méthacrylamide, le N-isopropyl méthacrylamide, le N-(2-hydroxy éthyl) méthacrylamide, le N-méthyl acrylamide, le N-éthyl acrylamide, le N-propyl acrylamide, le N-isopropyl acrylamide ou le N-(2-hydroxy éthyl) acrylamide.

Selon cet aspect plus particulier de la présente invention, le composé de formule (II) est le N-isopropyl acrylamide (dénommé ci-après NIPAM),

Selon un autre aspect particulier de la présente invention, ledit copolymère en peigne directement obtenu par le procédé tel que défini précédemment est caractérisé en ce que lesdits segments latéraux macromonomériques, sont issus de la polymérisation du composé de formule (II) telle que définie précédemment, dans laquelle, R₄ représente un atome d'hydrogène ou un radical méthyle et R₅ et R₆ représente chacun un radical méthyle ou un radical éthyle.

Selon cet aspect particulier le composé de formule (II) est plus particulièrement choisi parmi le N,N-diméthyl acrylamide, le N,N-diéthyl acrylamide, le N,N-diméthyl méthacrylamide ou le N,N-diéthyl méthacrylamide.

Par mélange tertio-butanol/eau, on désigne dans le procédé tel que défini ci-dessus un mélange dont la proportion volumique en eau est inférieure ou égal à 50%.

Grâce à son caractère cationique et ses propriétés thermo-épaississantes, le polyélectrolyte objet de la présente invention, est avantageusement utilisé comme épaississant et/ou comme émulsionnant dans des compositions cosmétiques ou pharmaceutiques destinées au soin et/ou au conditionnement des cheveux.

Le polyélectrolyte directement obtenu par le procédé tel que défini précédemment peut être formulé dans des formules cosmétiques ou pharmaceutiques comme des mousses, des gels, des lotions, des sprays, des shampoings, des conditionneurs, des lotions pour les mains et le corps, des écrans solaires, et plus généralement dans des produits de soin.

Dans le cas du traitement ou de l'entretien du cheveu, de telles compositions cosmétiques ou pharmaceutiques se présentent habituellement sous forme de shampoings d'émulsions, de microémulsions et notamment dans le cas des conditionneurs, d'émulsions vaporisables.

Une composition cosmétique ou pharmaceutique contenant comme agent épaississant, une quantité efficace du copolymère en peigne obtenu directement par le procédé tel que défini précédemment en contient une proportion pondérale comprise entre environ 0,1% et environ 5% en poids du polyélectrolyte tel que définie précédemment.

### Exemple 1 de synthèse du copolymère ATBS(NH4) / DMAPAM / NIPAM (rapport molaire : 70,6 / 7,8 / 21,6)

### (1) Préparation d'un télomère poly(N-isopropyl acrylamide)

On dissout à 25°C dans un réacteur thermostaté, 60g de N-isopropyl acrylamide (NIPAM) dans 75g d'un mélange tertio-butanol-eau (50/50 en volume), que l'on agite sous barbotage d'azote pendant environ 1 heure. On ajoute ensuite 0,9g de du chlorhydrate de 2-amino éthanethiol (AET.HCI). La polymérisation est initiée en ajoutant 1,33g de peroxyde de dilauroyle en portant la température à 60°C, puis on laisse le milieu réactionnel sous agitation et barbotage d'azote pendant encore 3 heures et demi. 87g de tertio-butanol sont ensuite ajouter pour conduire à un mélange réactionnel final blanc et pâteux.

### (2) Préparation du macromonomère

On ajoute au milieu réactionnel obtenu à l'étape (1) maintenu à une température de 10°C, 0,5g d'une solution de soude à 48% en poids, dans le tertio-butanol, pour amener le pH autour de 12 pendant. 3,16g de méthacrylate de glycidyle sont alors ajoutés et la réaction est laissée se dérouler pendant une heure. En fin de réaction, on ajoute environ 1,3 g d'acide chlorhydrique à 15% dans l'eau pour abaisser le pH à une valeur comprise entre 7 et 8.

La solution obtenue comprend 28,1% en poids de macromonomère NIPAM et 17% en poids d'eau.

### (3) Synthèse du copolymère en peigne

On dilue 40,5 g de la solution obtenue à l'étape 2 dans 487,5g de tertio-butanol dans le réacteur thermostaté à 25°C; On y ajoute 67,7 g de poudre d'acide 2-acrylamido 2-méthyl propanesulfonique, 5,7g de N-[3-(diméthylamino propyl)] acrylamide (DMAPAM; 4,95 g d'ammoniac sont injectés dans le mélange réactionnel de façon à obtenir un pH environ égal à 6. On ajoute alors 0,5 g d'eau et 0,48 g de triacrylate de triméthylol propane (TMPTA). Après barbotage d'azote pendant 1 h et chauffage à 60°C, la polymérisation est amorcée avec 1g de peroxyde de dilauroyle. Après 3h de réaction, le produit est vidangé, filtré et séché. Le copolymère en peigne recherché est obtenu sous forme d'une poudre blanche [dénommé dans les exemples suivants copolymère (1)].

Viscosité d'une solution à 1% du copolymère peigne dans l'eau 25°C :
µ = 10.000 mPas (Brookfield RVT Mobile 4; Vitesse :5 tours / minute)

Viscosité d'une solution à 1% du copolymère peigne dans l'eau 50°C :
µ = 50 000 mPas (Brookfield RVT Mobile 5; Vitesse :5 tours / minute)

Cette différence de viscosité constatée entre 25°C et 50°C, est caractéristique d'un polymère thermosensible.

### Exemple 2 : Soin antistress pour cheveux

### Formule

**Phase A**

| | |
|---|---|
| Eau : | QSP 100 % |
| Gomme xanthane | 0,50 % |

**Phase B**

| | |
|---|---|
| SEPICAP™ MP: | 3,00 % |

**Phase C**

| | |
|---|---|
| Copolymère 1 : | 4,00 % |

**Phase D**

| | |
|---|---|
| Butylène Glycol : | 5,00 % |
| LANOL™ 99 : | 5,00 % |
| SEPICIDE™ HB : | 0,30 % |
| SEPICIDE™ Cl : | 0,20 % |
| Parfum | 0,20 % |

### Mode Opératoire

Disperser la gomme xanthane dans l'eau avec une défloculeuse. Ajouter ensuite SEPICAP™ MP, puis le composé 1 ; le disperser puis ajouter les ingrédients de la phase D.

### Exemple 3 : Masque crème restructurant pour cheveux stressés et fragilisés

### Formule

**Phase A**

| | |
|---|---|
| MONTANOV™ 82 : | 3,00 % |
| LANOL™ P : | 6,00 % |
| AMONYL™ DM : | 1,00 % |
| Isononanoate d'isostéaryle : | 5,00 % |
| Copolymère 1 : | 2,50 % |

**Phase B**

| | |
|---|---|
| Eau : | QSP 100 % |

**Phase C**

| | |
|---|---|
| SEPICAP™ MP : | 3,00 % |
| SEPICIDE™ HB : | 0,30 % |
| SEPICIDE™ Cl : | 0,20 % |

### Mode Opératoire

Fondre la phase A à 75°C. Chauffer la phase B à 75°C. Emulsionner A dans B. Vers 40°C introduire les constituants de la phase C.

### Exemple 4 : Gel purifiant pour visage

### Formule

**Phase A**

| | |
|---|---|
| MONTALINE™ C 40 : | 7,00 % |
| Base nacrante 2078 : | 5,00 % |
| Copolymère 1 : | 2,00% |

**Phase B**

| | |
|---|---|
| Eau : | QSP 100 % |

### Exemple 5 : Shampoing colorant

### Formule

**Phase A**

| | |
|---|---|
| MONTALINE™ C 40 : | 15,00 % |
| Cocamphoacétate disodé : | 5,00 % |
| Cetrimonium chloride : | 1,00 % |
| SEPIPERL™ N : | 3,00 % |
| Copolymère 1 : | 3,00 % |

**Phase B**

| | |
|---|---|
| Couleur | QSP |
| Eau | QSP 100 % |

### Exemple 6 : Emulsion fluide à pH alcalin

| | |
|---|---|
| MARCOL™ 82 : | 5,0% |
| Hydroxyde sodium : | 10,0% |
| Eau : | q.s.p. 100% |
| Copolymère 1 : | 1,5% |

### Exemple 7 : Masque crème "rince off" restructurant pour cheveux stressés et fragilisés

| | |
|---|---|
| KETROL™T : | 0,5% |
| PECOSIL™ SPP50 : | 0,75% |
| N-cocoyl aminoacides : | 0,70% |
| Butylèneglycol : | 3,0% |
| Copolymère 1 : | 3,0% |
| MONTANOV™ 82 : | 3,0% |
| Huile de jojoba : | 1,0% |
| LANOL™ P : | 6,0% |
| AMONYL™ DM : | 1,0% |
| LANOL™ 99 : | 5,0% |
| SEPICIDE™ HB : | 0,3% |
| SEPICIDE™CI : | 0,2% |
| Parfum : | 0,2% |
| Eau : | qsp. 100% |

### Exemple 8 : Lotion capillaire

| | |
|---|---|
| Butylène glycol : | 3,0% |
| Copolymère 1 : | 3,0% |
| SIMULSOL™1293 : | 3,0% |
| Acide lactique : | qs. pH = 6 |
| SEPICIDE™ HB : | 0,2% |
| SEPICIDE™CI : | 0,3% |
| Parfum : | 0,3% |
| Eau : | qs. 100% |

### Exemple 9 : Shampooing protecteur et relaxant

| | |
|---|---|
| Amonyl™ 675 SB : | 5,0% |
| Sodium lauroyl éther sulfate à 28% : | 35,0% |
| Copolymère 1 : | 3,0% |
| SEPICIDE™ HB: | 0,5% |
| SEPICIDE™CI : | 0,3% |
| Hydroxyde de sodium : | q.s. pH = 7,2 |
| Parfum : | 0,3% |
| Colorant (FDC bleu 1/jaune 5) : | q.s. |
| Eau : | qsp. 100% |

### Exemple 10 : Protecteur "leave-on" ; Soin antistress pour cheveux

| | |
|---|---|
| KETROL™T : | 0,5% |
| Mélange de cocoyl aminoacides : | 3,0% |
| Butylèneglycol : | 5,0% |
| DC 1501 : | 5,0% |
| Copolymère 1 : | 4,0% |
| SEPICIDE™ HB: | 0,5% |
| SEPICIDE™CI : | 0,3% |
| Parfum : | 0,3% |
| Eau : | qsp.100 |

Les définitions des produits commerciaux utilisés dans les exemples sont les suivantes:
MONTALINE™ C40 : (cocamoniumcarbamoyl chloride) commercialisé par SEPPIC.
SEPIPERL™ N : (cocoyl glucoside *1* cocoyl alcohol) commercialisé par SEPPIC.
AMONYLT^{M} DM : (Quatemium 82) commercialisé par SEPPIC.
SEPICAP™ MP : (Sodium cocoyl amino acids *1* potassium dimethicone copolyol panthenyl phosphate) commercialisé par SEPPIC.
SIMULSOL™ 1293 est de l'huile de castor hydrogénée et éthoxylée, avec un indice d'éthoxylation égal à 40, commercialisé par la société SEPPIC.
KETROL™ T est de la gomme de xanthane commercialisée par la société KELCO.
LANOL™ 99 est de l'isononanoate d'isononyle commercialisé par la société SEPPIC. DC1501 est un mélange de cyclopentasiloxane et de diméthiconol commercialisé par la société DOW CHEMICAL.
MONTANOV™ 82 est un agent émulsionnant à base d'alcool cétéarylique et de cocoylglucoside.
Le SEPICIDE™ CI, imidazolidine urée, est un agent conservateur commercialisé par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
Le LANOL™ P est un additif à effet stabilisant commercialisé par la société SEPPIC.

## Revendications

1. Procédé de préparation d'un copolymère en peigne constitué d'un squelette polymérique sur lequel sont greffés des segments latéraux macromonomériques, dans lequel ledit squelette polymérique est constitué, pour 100% molaire d'unités monomériques constitutives dudit copolymère en peigne :
De plus de 55% jusqu'à 90% molaires, et plus particulièrement de 60% à 80% molaire, d'unités monomériques issues de l'acide 2-méthyl 2-[(1-oxo 2-propènyl) amino] 1-propanesulfonique (dénommé ci-après ATBS), libre, partiellement ou totalement salifié ;
De 1% à 20% molaire, plus particulièrement de 4% à 15% molaire, soit d'unités monomériques issues d'un monomère cationique soit, d'unités monomériques issues d'un monomère de formule (I) :
CH₂=C(R₁)-C(=O)-Y-(CH₂)ₘ-N(R₂)(R₃) (I)
dans laquelle m représente un nombre compris entre 1 et 4, Y représente O ou NH, R₁ représente un atome d'hydrogène ou un radical méthyle, R₂ et R₃ identiques ou différents représentent un radical méthyle ou un radical éthyle ;
- Optionnellement de 0,005% molaire à 1%, plus particulièrement de 0,01% molaire à 0,5% molaire d'unité monomériques issues d'au moins un monomère de réticulation comprenant au moins deux double liaisons carbone - carbone,
dans lequel lesdits segments latéraux macromonomériques sont issus, pour 100% molaire constitutives dudit copolymère en peigne, de la polymérisation de 9% à 30% molaire et plus particulièrement de 15% à 25% molaire, de N-alkyl acrylamide ou de N,N-dialkyl acrylamide) formule (II) :
CH₂=C(R₄)-C(=O)-N(R₅)(R₆) (II),
dans laquelle, R₄ représente un atome d'hydrogène ou un radical méthyle et R₅ et R₆ représente indépendamment l'un de l'autre, un atome d'hydrogène ou un radical alkyle comportant de 1 à 3 atomes de carbone éventuellement substitué par un groupe hydroxy, étant entendu qu'au moins un des radicaux R₅ ou R₆ ne représente pas un atome d'hydrogène, ledit procédé les étapes successives suivantes :
- Une étape (a) de réaction du composé de formule (II) :
CH₂=C(R₂)-C(=O)-N(R₃)(R₄) (II),
dans laquelle R₂ représente un atome d'hydrogène ou un groupement méthyle, R₃ un atome d'hydrogène ou un radical alkyle linéaire ou ramifié comportant de 1 à 4 atomes de carbone et R₄, identique ou différent de R₃, représente un radical alkyle linéaire ou ramifié comportant de 1 à 4 atomes de carbone, avec un composé limiteur de chaîne de formule (IVa) :
HS-R₁-NH₂ (IVa)
ou de formule (IVb) :
HS-R₁-C(=O)-OH (IVb)
formule (IVa) ou (IVb) dans lesquelles R₁ représente un radical divalent comportant de 1 à 4 atomes de carbone, en présence d'un initiateur de polymérisation, dans un mélange tertio-butanol / eau, pour obtenir un télomère poly(N-alkyl acrylamide) ou poly(N,N-dialkyl acrylamide), de formule (Va) :
H₂N-R₁-S-{CH₂-C(R₂)[C(=O)N(R₃)(R₄)]-}ₙ-H (Va)
ou de formule (Vb) :
HO-C(=O)-R₁-S-{CH₂-C(R₂)[C(=O)N(R₃)(R₄)]-}ₙ-H (Vb)
formule (Va) ou (Vb) dans lesquelles n représente un nombre entier supérieur ou égal à 2 et inférieur ou égal à 100 ; optionnellement
- Une étape (b) d'isolation et/ou de séchage dudit télomère de formule (Va) ou de formule (Vb) obtenu à l'étape (a) ;
- Une étape (c) d'obtention d'une solution du macromonomère de formule (VIIa) :
CH₂=C(R₅)-C(=O)-NH-R₁-Z-{CH₂-C(R₂)[C(=O)N(R₃)(R₄)]-}ₙ-H (VIIa) ;
ou de formule (Vllb) :
CH₂=C(R₅)-C(=O)-O-(C=O)-R₁-Z-{CH₂-C(R₂)[C(=O)N(R₃)(R₄)]-}ₙ-H (VIIb),
en partant du télomère de formule (Va) ou de formule (Vb) obtenu à l'étape (a), ou optionnellement à l'étape (b),
optionnellement
- Une étape (d) d'isolation et/ou de séchage dudit macromonomère de formule (VIIa) ou de formule (Vllb) obtenu à l'étape (c) ;
- Une étape (e) de copolymérisation dans le tertio-butanol, dudit macromonomère de formule (VIIa) ou de formule (Vllb) issu de l'étape (c) ou de l'étape (d), avec l'acide 2-acrylamido 2-méthyl propanesulfonique et soit le monomère cationique soit, le monomère de formule (I) telle que définie précédemment et/ou le ou les monomères de réticulation comprenant au moins deux double liaisons carbone-carbone dans les proportions molaires souhaitées, et si désiré,
- Une étape (f) de purification du copolymère peigne obtenu à l'étape (e) ;
**ledit procédé étant caractérisé en ce que**
- l'étape (c) est réalisée dans le tertio-butanol avec le méthacrylate de glycidyle (VI) et dans un rapport molaire (VI) / (IVa) ou VI / (IVb) inférieur ou égal à 10 et supérieur ou égal à 1 tout en maintenant le pH du milieu réactionnel à une valeur supérieure à 10 et inférieure ou égale à 13 plus particulièrement supérieure ou égale à 11 et inférieure ou égale à 12, pour obtenir, après ajustement du pH à une valeur supérieure ou égale à 7 et inférieure ou égale à 9, de préférence inférieure ou égale à 8, ladite solution du macromonomère de formule (VIIa) ou de formule (Vllb).

2. Procédé tel que défini à la revendication 1, **caractérisé en ce que**, lorsque ledit squelette polymérique comporte des unités monomériques issues du monomère de formule (I), celles-ci sont issues du :
- Méthacrylate de diméthyl amino éthyle ou du
- N-[3-(diméthylamino propyl)] acrylamide.

3. Procédé tel que défini à la revendication 1, **caractérisé en ce que**, lorsque ledit squelette polymérique comporte des unités monomériques issues d'un monomère cationique, celles-ci sont issues d'un monomère cationique de formule (III) :
CH₂=C(R₇)-C(=O)-Y-(CH₂)ₙ-N⁺(R₈)(R₉)(R₁₀), X⁻ (III)
dans laquelle n représente un nombre compris entre 1 et 4, Y représente O ou NH, R₇ représente un atome d'hydrogène ou un radical méthyle, R₈ et R₉ et R₁₀ identiques ou différents représentent un radical méthyle ou un radical éthyle et l'anion X⁻ un anion choisi parmi les ions bromure ou chlorure.

4. Procédé tel que défini à la revendication 3, **caractérisé en ce que**, lorsque ledit squelette polymérique comporte des unités monomériques issues du monomère cationique de formule (III), celles-ci sont issues des sels d'ammonium quaternaires suivants :
- Le sel de N,N,N-triméthyl 3-[(2-méthyl 1-oxo 2-propènyl) amino] propanammonium ;
- Le sel de N,N,N-triméthyl 3-[(1-oxo 2-propènyl) amino] propanammonium ;
- Le sel de N,N,N-triméthyl 2-[(1-0x0 2-propènyl) oxy] éthanammonium ; ou
- Le sel de N,N,N-triméthyl 2-[(2-méthyl 1-oxo 2-propènyl) oxy] éthanammonium,

5. Procédé tel que défini à la revendication 4, **caractérisé en ce que**, lorsque ledit squelette polymérique comporte des unités monomériques issues du monomère cationique de formule (III), celles-ci sont issues des sels d'ammonium quaternaires suivants :
- Le chlorure de N,N,N-triméthyl 3-[(1-oxo 2-propènyl) amino] propanammonium, ou
- Le chlorure de N,N,N-triméthyl 2-[(2-méthyl 1-oxo 2-propènyl) oxy] éthanammonium.

6. Procédé tel que défini à l'une quelconque des revendications 1 à 5, **caractérisé en ce que** lesdits segments latéraux macromonomériques sont issus de la polymérisation du composé de formule (II) telle que définie précédemment, dans laquelle, R₄ représente un atome d'hydrogène ou un radical méthyle et R₅ un atome d'hydrogène et R₆ représente un radical alkyle comportant de 1 à 3 atomes de carbone éventuellement substitué par un groupe hydroxy,

7. Procédé tel que défini à la revendication 6, **caractérisé en ce que** le composé de formule (II) est plus particulièrement choisi parmi le N-méthyl méthacrylamide, le N-éthyl méthacrylamide, le N-propyl méthacrylamide, le N-isopropyl méthacrylamide, le N-(2-hydroxy éthyl) méthacrylamide, le N-méthyl acrylamide, le N-éthyl acrylamide, le N-propyl acrylamide, le N-isopropyl acrylamide ou le N-(2-hydroxy éthyl) acrylamide.

8. Procédé tel que défini à la revendication 7, **caractérisé en ce que** le composé de formule (II) est le N-isopropyl acrylamide.

9. Procédé tel que défini à l'une quelconque des revendications 1 à 5, **caractérisé en ce que** lesdits segments latéraux macromonomériques, sont issus de la polymérisation du composé de formule (II) telle que définie précédemment, dans laquelle, R₄ représente un atome d'hydrogène ou un radical méthyle et R₅ et R₆ représente chacun un radical méthyle ou un radical éthyle.

10. Procédé tel que défini à la revendication 9, **caractérisé en ce que** le composé de formule (II) est choisi parmi le N,N-diméthyl acrylamide, le N,N-diéthyl acrylamide, le N,N-diméthyl méthacrylamide ou le N,N-diéthyl méthacrylamide.

## Patentansprüche

1. Verfahren zur Vorbereitung eines Kamm-Copolymers bestehend aus einem Polymerskelett, auf dem die makromonomerischen lateralen Segmente aufgepfropft sind, wobei das Polymerskelett zu 100 Mol-% aus monomerischen Einheiten, die für das Kamm-Copolymer konstitutiv sind, besteht:
von mehr als 55 Mol-% bis 90 Mol-%, und insbesondere von 60 Mol-% bis 80 Mol-% aus monomerischen Einheiten aus 2-Methyl 2-[(1-Oxo-2-Propenyl) Amino] 1-Propansulfonsäure (im Weiteren ATBS), frei, teilweise oder komplett als Salz;
von 1 Mol-% bis 20 Mol-%, insbesondere von 4 Mol-% bis 15 Mol-% entweder aus monomerischen Einheiten aus einem kationischen Monomer oder aus monomerischen Einheiten aus einem Monomer der Formel (I):
CH₂=C(R₁)-C(=O)-Y-(CH₂)ₘ-N(R₂)(R₃) (I)
wobei m eine Zahl zwischen 1 und 4 darstellt, Y O oder NH darstellt, R₁ ein Wasserstoffatom oder ein Methylradikal darstellt, R₂ und R₃, ob gleich oder unterschiedlich, ein Methyl- oder ein Ethylradikal darstellen;
- optional von 0,005 Mol-% bis 1 Mol-%, insbesondere von 0,01 Mol-% bis 0,5 Mol-%, aus monomerischen Einheiten aus mindestens einem vernetzenden Monomer, das mindestens zwei Kohlenstoff-Kohlenstoff-Doppelbindungen umfasst,
wobei die lateralen makromonomerischen Segmente, zu 100 Mol-% konstitutiv für das Kamm-Copolymer, aus der Polymerisation von 9 Mol-% bis 30 Mol-% und insbesondere von 15 Mol-% bis 25 Mol-% von N-Alkylacrylamid oder N,N-Dialkylacrylamid) Formel (II) stammen:
CH₂=C(R₄)-C(=O)-N(R₅)(R₆) (II),
wobei R₄ ein Wasserstoffatom oder ein Methylradikal und R₅ und R₆ unabhängig voneinander ein Wasserstoffatom oder ein Alkylradikal darstellen mit 1 bis 3 Kohlenstoffatomen, die eventuell durch eine Hydroxygruppe ersetzt werden, wobei es sich versteht, dass mindestens eines der Radikale R₅ oder R₆ kein Wasserstoffatom darstellt und es bei diesem Verfahren folgende aufeinanderfolgende Schritte gibt:
- einen Reaktionsschritt (a) der Verbindung der Formel (II):
CH₂=C(R₂)-C(=O)-N(R₃)(R₄) (II),
wobei R₂ ein Wasserstoffatom oder eine Methylgruppierung darstellt und R₃ ein Wasserstoffatom oder ein lineares oder verzweigtes Alkylradikal mit 1 bis 4 Kohlenstoffatomen und R₄, gleich oder unterschiedlich von R₃, ein lineares oder verzweigtes Alkylradikal mit 1 bis 4 Kohlenstoffatomen darstellt mit einer kettenbegrenzenden Verbindung der Formel (IVa):
HS-R₁-NH2 (IVa)
oder der Formel (IVb):
HS-R₁-C(=O)-OH (IVb)
Formel (IVa) oder (IVb), wobei R₁ ein zweiwertiges Radikal mit 1 bis 4 Kohlenstoffatomen darstellt, unter Anwesenheit eines Polymerisationsinitiators, in einer Mischung tert-Butanol / Wasser, um ein Telomer Poly(N-Alkyl Acrylamid) oder Poly(N,N-Dialkyl Acrylamid) folgender Formel (Va) zu erhalten:
H₂N-R₁-S-{CH₂-C(R₂)[C(=O)N(R₃)(R₄)-}ₙ-H (Va)
oder der Formel (Vb):
HO-C(=O)-R₁-S-{CH₂-C(R₂)[C(=O)N(R₃)(R₄)-}ₙ-H (Vb)
Formel (Va) oder (Vb), wobei n eine ganze Zahl darstellt, die höher oder gleich 2 ist und geringer oder gleich 100 ist; optional
- einen Isolierungsschritt (b) und/oder einen Trocknungsschritt des Telomers der Formel (Va) oder der Formel (Vb) erhalten in Schritt (a);
- einen Schritt (c) zum Erhalt einer Makromonomerlösung der Formel (Vlla):
CH₂=C(R₅)-C(=O)-NH-R₁-Z-{CH₂-C(R₂)[C(=O)N(R₃)(R₄)]-}ₙ-H (Vlla);
oder der Formel (Vllb):
CH₂=C(R₅)-C(=O)-O-(C=O)-R₁-Z-{CH₂-C(R₂)[C(=O)N(R₃)(R₄)]-}ₙ-H (VIIb),
ausgehend vom Telomer der Formel (Va) oder der Formel (Vb) erhalten in Schritt (a), oder optional in Schritt (b),
optional
- einen Isolierungsschritt (d) und/oder einen Trocknungsschritt des Makromonomers der Formel (Vlla) oder der Formel (Vllb) erhalten in Schritt (c);
- einen Copolymerisationsschritt (e) im tert-Butanol, des Makromonomers der Formel (Vlla) oder der Formel (Vllb) aus Schritt (c) oder Schritt (d), mit 2- Acrylamid 2-Methylpropansulfonsäure und entweder dem kationischen Monomer oder dem Monomer der Formel (I) wie vorher definiert und/oder dem oder den vernetzenden Monomeren mit mindestens zwei Kohlenstoff-Kohlenstoff-Doppelbindungen in den gewünschten Stoffmengenverhältnissen und falls erbeten,
- einen Reinigungsschritt (f) des Kamm-Copolymers erhalten in Schritt (e);
**wobei das Verfahren dadurch gekennzeichnet ist, dass**
- Schritt (c) im tert-Butanol mit Glycidylmethacrylat (VI) und in einem Stoffmengenverhältnis (VI) / (IVa) oder VI / (IVb) kleiner oder gleich 10 und größer oder gleich 1 unter Beibehaltung des pH-Werts des Reaktionsmediums auf einem Wert größer als 10 und kleiner oder gleich 13, insbesondere größer oder gleich 11 und kleiner oder gleich 12, erfolgt, um nach Anpassung des pH-Werts auf einen Wert größer oder gleich 7 und kleiner oder gleich 9, vorzugsweise kleiner oder gleich 8, die Makromonomerlösung der Formel (VIIa) oder der Formel (VIIb) zu erhalten.

2. Verfahren nach Anspruch 1, **dadurch gekennzeichnet, dass**, wenn das Polymerskelett monomerische Einheiten aus dem Monomer der Formel (I) umfasst, diese entstanden sind aus:
- Dimethylaminoethyl-Methacrylat oder aus
- N-[3-(Dimethylaminopropyl)] Acrylamid.

3. Verfahren nach Anspruch 1, **dadurch gekennzeichnet, dass**, wenn das Polymerskelett monomerische Einheiten aus einem kationischen Monomer umfasst, diese aus einem kationischen Monomer der Formel (III) entstanden sind:
CH₂=C(R₇)-C(=0)-Y-(CH₂)ₙ-N⁺(R₈)(R₉)(R₁₀), X⁻ (III)
wobei n eine Zahl zwischen 1 und 4 darstellt, Y O oder NH darstellt, R₇ ein Wasserstoffatom oder ein Methylradikal darstellt, R₈ und R₉ und R₁₀, egal ob gleich oder unterschiedlich, ein Methylradikal oder ein Ethylradikal darstellen und das Anion X⁻ ein Anion darstellt, das aus Bromid- oder Chloridionen gewählt wurde.

4. Verfahren nach Anspruch 3, **dadurch gekennzeichnet, dass**, wenn das Polymerskelett monomerische Einheiten aus einem kationischen Monomer der Formel (III) umfasst, diese aus den folgenden quartären Ammoniumsalzen entstanden sind:
- N,N,N-Trimethyl 3-[(2-Methyl 1-Oxo 2-Propenyl) Amino] Propanammonium-Salz;
- N,N,N-Trimethyl 3-[(1-Oxo 2-Propenyl) Amino] Propanammonium-Salz;
- N,N,N-Trimethyl 2-[(1-Oxo 2-Propenyl) Oxy] Ethanammonium-Salz; oder
- N,N,N-Trimethyl 2-[(2-Methyl 1-Oxo 2-Propenyl) Oxy] Ethanammonium-Salz.

5. Verfahren nach Anspruch 4, **dadurch gekennzeichnet, dass**, wenn das Polymerskelett monomerische Einheiten aus einem kationischen Monomer der Formel (III) umfasst, diese aus den folgenden quartären Ammoniumsalzen entstanden sind:
- N,N,N-Trimethyl 3-[(1-oxo 2-Propenyl) Amino] Propanammonium-Chlorid, oder
- N,N,N-Trimethyl 2-[(2-Methyl 1-Oxo 2-Propenyl) Oxy] Ethanammonium-Chlorid.

6. Verfahren nach einem der Ansprüche 1 bis 5, **dadurch gekennzeichnet, dass** die makromonomerischen lateralen Segmente aus der Polymerisation der Verbindung der Formel (II), wie vorher definiert, stammen, wobei R₄ ein Wasserstoffatom oder ein Methylradikal darstellt und R₅ ein Wasserstoffatom und R₆ ein Alkylradikal darstellt mit 1 bis 3 Kohlenstoffatomen eventuell ersetzt durch eine Hydroxygruppe.

7. Verfahren nach Anspruch 6, **dadurch gekennzeichnet, dass** die Verbindung der Formel (II) insbesondere ausgewählt wird aus N-Methylmethacrylamid, N-Ethylmethalcrylamid, N-Propylmethacrylamid N-Isopropylmethacrylamid, N-(2-Hydroxyethyl) Methacrylamid, N-Methylacrylamid, N-Ethylacrylamid, N-Propylacrylamid, N-Isopropylacrylamid oder N-(2-Hydroxyethyl) Acrylamid.

8. Verfahren nach Anspruch 7, **dadurch gekennzeichnet, dass** die Verbindung der Formel (II) N-Isopropyl Acrylamid ist.

9. Verfahren nach einem der Ansprüche 1 bis 5, **dadurch gekennzeichnet, dass** die makromonomerischen lateralen Segmente aus der Polymerisation der Verbindung der Formel (II), wie vorher definiert, stammen, wobei R₄ ein Wasserstoffatom oder ein Methylradikal darstellt und R₅ und R₆ jeweils ein Methylradikal oder ein Ethylradikal darstellen.

10. Verfahren nach Anspruch 9, **dadurch gekennzeichnet, dass** die Verbindung der Formel (II) aus N,N-Dimethyl Acrylamid, N,N-Diethyl Acrylamid, N,N-Dimethyl Methacrylamid oder N,N-Diethyl Methacrylamid ausgewählt wird.

## Claims

1. Method for preparing a comb polymer consisting of a polymer backbone on which lateral macromonomer segments are grafted, wherein said polymer backbone consists, for 100% molar of monomeric units constituting said comb polymer:
Of more than 55% and up to 90% molar, and more particularly from 60% to 80% molar, of monomeric units from 2-methyl 2-[(1-oxo 2-propenyl) amino] 1-propanesulphonic acid (hereinafter referred to as ATBS), free, partially or completely salified;
From 1% to 20% molar, more particularly from 4% to 15% molar, either of monomeric units from a cationic monomer or of monomeric units from a monomer of formula (I):
CH₂=C(R₁)-C(=O)-Y-(CH₂)ₘ-N(R₂)(R₃) (I)
wherein m represents a number between 1 and 4, Y represents O or NH, R₁ represents a hydrogen atom or a methyl radical, R₂ and R₃ identical or different represent a methyl radical or an ethyl radical;
- Optionally of 0.005% molar to 1%, more particularly from 0.01% molar to 0.5% molar of monomeric units from at least one cross-linking monomer comprising at least two carbon-carbon double bonds,
wherein said lateral macromonomer segments come, for 100% molar constituting said comb polymer, from the polymerisation from 9% to 30% molar and more particularly from 15% to 25% molar, of N-alkyl acrylamide or of N,N-dialkyl acrylamide) formula (II):
CH₂=C(R₄)-C(=O)-N(R₅)(R₆) (II),
wherein, R₄ represents a hydrogen atom or a methyl radical and R₅ and R₆ represent, independent of each other, a hydrogen atom or an alkyl radical comprising from 1 to 3 carbon atoms optionally substituted by a hydroxy group, with the understanding that at least one of the radicals R₅ or R₆ does not represent a hydrogen atom, said method comprising the following successive steps:
- A step (a) of reaction of the compound of formula (II):
CH₂=C(R₂)-C(=O)-N(R₃)(R₄) (II),
wherein R₂ represents a hydrogen atom or a methyl group, R₃ a hydrogen atom or a linear or branched alkyl radical comprising from 1 to 4 carbon atoms and R₄, identical or different from R₃, represents a linear or branched alkyl radical comprising from 1 to 4 carbon atoms, with a chain-limiting compound of formula (IVa):
HS-R₁-NH₂ (IVa)
or of formula (IVb):
HS-R₁-C(=O)-OH (IVb)
formula (IVa) or (IVb) wherein R₁ represents a divalent radical comprising from 1 to 4 carbon atoms, in the presence of a polymerisation initiator, in a tertio-butanol / water mixture, in order to obtain a poly(N-alkyl acrylamide) or poly(N,N-dialkyl acrylamide) telomere, of formula (Va):
H₂N-R₁-S-{CH₂-C(R₂)[C(=O)N(R₃)(R₄)]-}ₙ-H (Va)
or of formula (Vb):
HO-C(=O)-R₁-S-{CH₂-C(R₂)[C(=O)N(R₃)(R₄)]-}ₙ-H (Vb)
formula (Va) or (Vb) wherein n represents an integer greater than or equal to 2 and less than or equal to 100; optionally
- A step (b) of insolating and/or drying of said telomere of formula (Va) or of formula (Vb) obtained in the step (a);
- A step (c) of obtaining a solution of the macromonomer of formula (Vila):
CH₂=C(R₅)-C(=O)-NH-R₁-Z-{CH₂-C(R₂)[C(=O)N(R₃)(R₄)]-}ₙ-H (VIIa);
or of formula (VIIb):
CH₂=C(R₅)-C(=O)-O-(C=O)-R₁-Z-{CH₂-C(R₂)[C(=O)N(R₃)(R₄)]-}ₙ-H (VIIb),
using the telomere of formula (Va) or of formula (Vb) obtained in the step (a), or optionally in the step (b),
optionally
- A step (d) of insolating and/or drying said macromonomer of formula (Vila) or of formula (Vllb) obtained in the step (c);
- A step (e) of copolymerisation in tertio-butanol, of said macromonomer of formula (Vila) or of formula (Vllb) from the step (c) or from the step (d), with 2-acrylamido 2-methyl propanesulphonic acid and either the cationic monomer or the monomer of formula (I) such as defined hereinabove and/or the cross-linking monomer or monomers comprising at least two double carbon-carbon bonds in the molar proportions wanted, and if desired,
- A step (f) of purification of the comb copolymer obtained in the step (e);
**said method being characterised in that**
- the step (c) is carried out in tertio-butanol with glycidyl methacrylate (VI) and in a molar ratio (VI) / (IVa) or VI / (IVb) less than or equal to 10 and greater than or equal to 1 while still maintaining the pH of the reaction medium at a value greater than 10 and less than or equal to 13 more particularly greater than or equal to 11 and less than or equal to 12, in order to obtain, after adjusting the pH to a value greater than or equal to 7 and less than or equal to 9, preferably less than or equal to 8, said solution of the macromonomer of formula (Vila) or of formula (VIIb).

2. Method such as defined in claim 1, **characterised in that**, when said polymer backbone comprises monomeric units from the monomer of formula (I), the latter come from:
- Dimethylaminoethyl methacrylate or from
- N-[3-(dimethylaminopropyl)] acrylamide.

3. Method such as defined in claim 1, **characterised in that**, when said polymer backbone comprises monomeric units from a cationic monomer, the latter come from a cationic monomer of formula (III):
CH₂=C(R₇)-C(=O)-Y-(CH₂)ₙ-N⁺(R₈)(R₉)(R₁₀), X⁻ (III)
wherein n represents a number between 1 and 4, Y represents O or NH, R₇ represents a hydrogen atom or a methyl radical, R₈ and R₉ and R₁₀ identical or different represent a methyl radical or an ethyl radical and the anion X⁻ an anion chosen from the bromide or chloride ions.

4. Method such as defined in claim 3, **characterised in that**, when said polymer backbone comprises monomeric units from the cationic monomer of formula (III), the latter come from the following quaternary ammonium salts:
- N,N,N-trimethyl 3-[(2-methyl 1-oxo 2-propenyl) amino] propanammonium salt;
- N,N,N-trimethyl 3-[(1-oxo 2-propenyl) amino] propanammonium salt;
- N,N,N-trimethyl 2-[(1-oxo 2-propenyl) oxy] ethanammonium salt; or
- N,N,N-trimethyl 2-[(2-methyl 1-oxo 2-propenyl) oxy] ethanammonium salt.

5. Method such as defined in claim 4, **characterised in that**, when said polymer backbone comprises monomeric units from the cationic monomer of formula (III), the latter come from the following quaternary ammonium salts:
- N,N,N-trimethyl 3-[(1-oxo 2-propenyl) amino] propanammonium chloride, or
- N,N,N-trimethyl 2-[(2-methyl 1-oxo 2-propenyl) oxy] ethanammonium chloride.

6. Method such as defined in any of claims 1 to 5, **characterised in that** said lateral macromonomer segments come from the polymerisation of the compound of formula (II) such as defined hereinabove, wherein, R₄ represents a hydrogen atom or a methyl radical and R₅ a hydrogen atom and R₆ represents an alkyl radical comprising from 1 to 3 carbon atoms optionally substituted by a hydroxy group.

7. Method such as defined in claim 6, **characterised in that** the compound of formula (II) is more particularly chosen from N-methyl methacrylamide, N-ethyl methacrylamide, N-propyl methacrylamide, N-isopropyl methacrylamide, N-(2-hydroxy ethyl) methacrylamide, N-methyl acrylamide, N-ethyl acrylamide, N-propyl acrylamide, N-isopropyl acrylamide or N-(2-hydroxy ethyl) acrylamide.

8. Method such as defined in claim 7, **characterised in that** the compound of formula (II) is N-isopropyl acrylamide.

9. Method such as defined in any of claims 1 to 5, **characterised in that** said lateral macromonomer segments, come from the polymerisation of the compound of formula (II) such as defined hereinabove, wherein, R₄ represents a hydrogen atom or a methyl radical and R₅ and R₆ each represent a methyl radical or an ethyl radical.

10. Method such as defined in claim 9, **characterised in that** the compound of formula (II) is chosen from N,N-dimethyl acrylamide, N,N-diethyl acrylamide, N,N-dimethyl methacrylamide or N,N-diethyl methacrylamide.
